(19) Europäisches Patentamt
European Patent Office
Office européen des brevets

(11) EP 2 120 047 A1

(12) DEMANDE DE BREVET EUROPEEN

(43) Date de publication:
18.11.2009 Bulletin 2009/47

(51) Int Cl.:
G01N 30/46 (2006.01)        G01N 30/86 (2006.01)
G01N 33/28 (2006.01)        G01N 30/02 (2006.01)

(21) Numéro de dépôt: 09290305.3

(22) Date de dépôt: 24.04.2009

(84) Etats contractants désignés:
AT BE BG CH CY CZ DE DK EE ES FI FR GB GR
HR HU IE IS IT LI LT LU LV MC MK MT NL NO PL
PT RO SE SI SK TR

(30) Priorité: 14.05.2008 FR 0802637

(71) Demandeur: IFP
92852 Rueil-Malmaison Cédex (FR)

(72) Inventeurs:
• Bertoncini, Fabrice
69007 Lyon (FR)
• Celse , Benoît
69740 Genas (FR)
• Dartiguelongue, Cyril
69360 St. Symphorien d'Ozone (FR)

(54) **Procédé pour déterminer des propriétés physico-chimiques d'un échantillon pétrolier à partir de chromatographie en phase gazeuse bidimensionnelle**

(57)     - Procédé pour déterminer des propriétés physico-chimiques d'un échantillon pétrolier à partir de chromatographie en phase gazeuse bidimensionnelle.
- On détermine les quantités de *n* composés moléculaires présents dans l'échantillon, au moyen d'une chromatographie en phase gazeuse bidimensionnelle. Puis, on détermine au moins une propriété physique, tel que l'indice d'octane, à partir de ces quantités. Pour ce faire on utilise une relation préalablement calibrée, qui relie la propriété physique aux quantités.

Fig. 2

EP 2 120 047 A1

## Description

La présente invention concerne le domaine de l'analyse physique et chimique d'échantillons pétroliers. On appelle « *échantillon pétrolier* », un mélange d'hydrocarbures, c'est-à-dire un mélange de composés organiques contenant principalement des atomes de carbone (C) et d'hydrogène (H).

En particulier, l'invention propose une méthode de détermination des propriétés physicochimiques d'un échantillon pétrolier, à partir d'une analyse quantitative de sa composition moléculaire.

La fabrication de carburants commerciaux, l'optimisation et le contrôle de fabrication des différentes bases des pools carburants (essence, gazole ou kérosène) imposent le contrôle précis de propriétés physiques macroscopiques afin de se conformer aux spécifications du marché. On appelle propriétés macroscopiques, ou propriétés globales, les propriétés d'un échantillon pétrolier qui s'observent à l'échelle de l'échantillon, par opposition aux propriétés qui s'observent à une échelle inférieure (échelle du composé moléculaire). Les propriétés physiques macroscopiques sont par exemple l'indice d'octane, l'indice de cétane, la densité, etc. Le raffineur n'a pas systématiquement accès à la mesure de ces propriétés physiques, notamment lorsque la quantité de carburant disponible n'est pas suffisante pour réaliser la mesure. Le suivi et l'optimisation des unités industrielles de production des bases des pools carburants requièrent également, de plus en plus, une connaissance fine des mécanismes mis en jeu dans les réacteurs.

En outre, le raffineur souhaite pouvoir prédire l'impact d'une transformation des carburants sur les propriétés physiques de ce dernier. Par exemple, le spécialiste souhaite pouvoir simuler l'effet d'une modification de l'intervalle de distillation d'un échantillon, ou l'effet de la transformation chimique d'une famille de composés (hydrogénation des composés aromatiques en composés cycliques saturés) sur les propriétés telles que l'indice d'octane, l'indice de cétane ou la densité.

Dans cette optique, la connaissance des propriétés physiques macroscopiques des carburants, mais également la connaissance des propriétés par coupes, deviennent indispensables. Une coupe d'un échantillon pétrolier est le distillat obtenu entre deux températures déterminées au cours d'une opération de distillation. Les besoins des raffineurs sont ainsi de plus en plus orientés vers la mise au point de modèles de propriétés explicites vis-à-vis de la composition chimique des carburants.

## État de la technique

Des méthodes physiques ont été normalisées pour évaluer ces propriétés, mais elles sont généralement coûteuses en temps et exigeantes en volume d'échantillons nécessaires pour les mettre en oeuvre.

Pour des échantillons distillables entre 0˚C et 220˚C (coupes essences et naphtas), ces méthodes physiques ont été avantageusement remplacées, par l'utilisation de lois de mélanges des coupes fondées sur l'expérience des raffineurs, ou encore par la mise en oeuvre d'outils analytiques dédiés, permettant de relier la composition des produits à une propriété physique. Les méthodes d'analyse chimiques modernes comme la spectrophotométrie infrarouge (PIR) ou la chromatographie en phase gazeuse (CPG) ont ainsi permis : l'accès à une information moléculaire plus complète des coupes essences, et le développement de corrélations entre la composition chimique de l'essence obtenue par ces techniques d'analyses, et plusieurs propriétés dont l'indice d'octane, la densité, le pouvoir calorifique, etc. Les premiers travaux ont été réalisés à partir de l'analyse par chromatographie phase gaz (CPG), et sont décrits dans le document suivant : Jenkins G.J., Mc Taggart N.G., Watkin B.L.H., (1968) «GLC for on-stream Octane number Rating of stabilized catalitic reformates», Gas Chromatography, Ed. Inst. Petr., London, p 185-198.

Des modèles obtenus par régression linéaire ont été élaborés. Ils regroupent les données chromatographiques (concentration massique des constituants individuels (composés)) en une trentaine de groupes de constituants par analogie de structure chimique. Dans le début des années 80 sont apparues les premières publications concernant le calcul des indices d'octane à partir de la spectrométrie proche infra-rouge : Kelly J.J., Barlow C.H., Jinguji T.M. et Callis J.B. (1989) «Prediction of octane numbers from near-infrared spectral features in the range 660-1212 nm», Analytical Chemistry, Vol 61, No 4, 313. Et les travaux de D. Lambert et al. ont marqué le début de l'utilisation de cette technique en outil de contrôle du pool essence des raffineries : Espinosa A., Lambert D. et Valleur M. (1995) « Use NIR technology to optimize plant operation, Hydrocabon Processing », february, 86. Suivant la même approche, J.P.Durand, Y. Boscher, N. Petroff and M. Berthelin, J. Chromatogr., 395 (1987) 229, ont montré qu'il est possible de décrire la masse volumique, la densité, la tension de vapeur REID, etc. à partir d'une analyse compositionnelle de l'essence.

Pour des échantillons distillables entre 220˚C et450˚C (cas des coupes gazoles et distillats moyens), d'autres techniques ont dû être mises au point. En effet, un tel détail compositionnel n'est pas disponible pour les coupes gazoles ou les distillats moyens qui présentent des limitations sévères pour les méthodes d'analyse évoquées précédemment. Il est en effet connu des spécialistes, que l'augmentation du nombre d'isomères avec le nombre d'atomes de carbone, rend illusoire l'extension de ce degré d'information à des coupes plus lourdes que les essences. Pour la CPG, le manque de pouvoir séparatif empêche de disposer d'une distribution des hydrocarbures par familles. Ceci rend inopérant les modèles de prédiction de propriétés basés sur une description moléculaire. Seule la distribution massique des composants en fonction du point d'ébullition est accessible. Pour la spectrométrie de masse, seule une information moléculaire plus sommaire est disponible (obtenue selon la méthode dite de Fitzgerald (référencée ASTMD2425)). Cependant cette méthode n'est applicable qu'à des échantillons ayant une gamme d'intervalles de distillation bien établie (points initiaux et finaux figés, fraction distillable d'au moins 70˚C dans l'intervalle), ou des teneurs limitées en oléfines (2 % m/m au maximum). En outre, elle ne permet pas de quantifier séparément les paraffines linéaires et ramifiées, ce qui ne permet pas de déterminer certaines propriétés fortement dépendantes du taux de ramification (indice de cétane, densité, etc.).

Les spécialistes combinent alors les données obtenues par CPG, SM ou un autre détecteur informatif (comme le détecteur par émission atomique- AED) pour contourner les limitations intrinsèques à chacune des techniques et prédire les propriétés macroscopiques des coupes kérosènes ou distillats moyens.

Ainsi, on connaît par le brevet US 5 699 269 une méthode qui permet de prédire les propriétés macroscopiques de coupes pétrolières à partir d'une analyse de chromatographie en phase gazeuse couplée à la spectrométrie de masse (CPG/MS). Cette méthode suppose que la base de calibration du modèle de propriétés contient des échantillons dont la composition est proche de celle de l'échantillon à analyser. Cette invention permet de prédire les propriétés macroscopiques des coupes pétrolières, mais n'est pas applicable à la prédiction de propriétés de simulation de coupes ou de simulation de transformations chimiques. En d'autres termes, cette méthode n'est pas extrapolable en dehors de sa base de calibration. Elle ne répond pas au besoin de simulation de propriétés des carburants car elle n'est pas basée sur le lien propriétés-composition chimique fine des carburants.

Dans le même esprit, le brevet US 6 275 775 décrit une méthode de prédiction de propriétés de fractions pétrolières à l'aide de la chromatographie en phase gazeuse couplée à un détecteur à émission atomique (CPG-AED). Les propriétés prédites sont macroscopiques, mais également fonction de l'intervalle de distillation de l'échantillon. Cette invention permet donc de déduire de l'analyse de CPG-AED les profils de propriétés des gazoles en fonction de la courbe de distillation. En revanche, elle ne permet pas de simuler une transformation chimique de coupe pétrolière (par exemple la transformation de composés aromatiques en composés cycliques saturés par procédé d'hydrotraitement).

L'approche par spectrophotométrie proche infrarouge a également été mise en oeuvre sur des distillats moyens. Cependant, cette méthode est corrélative et est très dépendante de la représentativité de la base de données. En outre, elle n'est pas applicable à la prédiction de propriétés de simulation de coupes ou de simulation de transformations chimiques. En d'autres termes, cette méthode n'est pas extrapolable en dehors de sa base de calibration. Elle ne répond pas au besoin de simulation de propriétés des carburants car elle n'est pas basée sur le lien propriétés-composition chimique fine des carburants.

En conclusion, les méthodes connues des spécialistes reposent sur des modèles corrélatifs, et non sur des modèles explicatifs, dans la mesure où ils ne sont pas fondés sur le détail moléculaire des fractions pétrolières analysées. En particulier, les méthodes analytiques conventionnelles d'analyse des échantillons distillables entre 150 et 450˚C, comme les coupes gazoles, n'offrent pas un détail analytique suffisant pour permettre de modéliser les propriétés d'usage de ces échantillons. Le point clé pour la prédiction de propriétés réside dans la relation étroite entre la propriété à prédire et la composition chimique détaillée (analyse par familles et par nombres d'atomes de carbones par exemple pour les produits pétroliers).

L'objet de l'invention est un procédé alternatif de détermination des propriétés physicochimiques macroscopiques d'un échantillon pétrolier, permettant de s'affranchir des limitations des méthodes conventionnelles. Le procédé y parvient en évaluant ces propriétés à partir de données quantitatives issues d'une chromatographie gazeuse bidimensionnelle. Cette analyse permet non seulement de prédire les propriétés macroscopiques d'un échantillon, mais également d'estimer ces propriétés pour une coupe pétrolière quelconque, et d'estimer l'évolution de ces propriétés suite à des transformations chimiques de l'échantillon pétrolier.

**Le procédé selon l'invention**

L'invention concerne un procédé de détermination d'au moins une propriété physique d'un mélange de composés moléculaires tel qu'un échantillon pétrolier, **caractérisé en ce que** l'on réalise les étapes suivantes :

-    on détermine les quantités de $n$ composés présents dans ledit mélange, au moyen d'une chromatographie en phase gazeuse bidimensionnelle ;

-    on détermine ladite propriété physique à partir desdites quantités, au moyen d'une relation $f$ préalablement calibrée reliant ladite propriété physique auxdites quantités.

Selon l'invention, la relation $f$ peut être définie par : $P_m = f(\alpha_i, Q_i, n)$, où $P_m$ est la propriété physique, $n$ est le nombre de composés moléculaires séparés par la chromatographie, $\alpha_i$ est un coefficient à calibrer et associé à un composé $i$, et $Q_i$ est la quantité du composé $i$ déterminée par la chromatographie. On peut alors calibrer cette relation $f$ en réalisant les étapes suivantes :

- on détermine des quantités de composés présents dans au moins un second mélange contenant au moins les $n$ composés moléculaires, au moyen d'une chromatographie en phase gazeuse bidimensionnelle ;

- on mesure ladite propriété physique $P_m$ dudit second mélange ;

- on initialise les coefficients $\alpha_i$ en leur affectant une valeur connue de propriété physique pour un échantillon ne contenant que le composé $i$ ;

- on calcule ladite propriété physique au moyen de ladite fonction $f$ ;

- on modifie les coefficients $\alpha_i$ de façon à minimiser une différence entre la valeur de la propriété physique calculée, et la valeur de la propriété physique mesurée.

On peut par exemple définir la relation $f$ par : $f(\alpha_i,\ Q_i,\ n) = \sum_{i=1}^{i=n} \alpha_i \cdot Q_i$

On peut réaliser la chromatographie en phase gazeuse bidimensionnelle, au moyen des étapes suivantes :

- on enregistre un signal chromatographique comportant des pics chromatographiques ;

- on génère un chromatogramme en deux dimensions dont chaque colonne correspond à une portion dudit signal chromatographique, lesdits pics chromatographiques formant des taches sur ledit chromatogramme ;

- on délimite lesdites taches à l'aide de polygones ;

- on ajuste lesdits polygones :

  - en identifiant des portions dudit signal chromatographique comprises entre deux intersections dudit polygone avec des colonnes dudit chromatogramme ;

  - en déterminant des temps de début, des temps de fin et des maximums de pics chromatographiques présents sur lesdites portions ;

  - en déplaçant lesdits points d'intersection en fonction desdits temps de début, desdits temps de fin et desdits maximums de pics chromatographiques ;

- on détermine une quantité d'au moins un composé moléculaire en calculant l'aire dudit polygone ainsi ajusté.

Selon l'invention, on peut déterminer la quantité d'un composé en estimant sa concentration à partir de l'aire du polygone.

La propriété physique peut être choisie parmi les propriétés suivantes :

- propriétés de combustion : indice d'octane, indice de cétane, point de fumée et densité ;

- propriétés à froid : température limite de filtrabilité, point de trouble, point d'écoulement, température de disparition des cristaux.

Selon l'invention, le mélange peut être avantageusement un échantillon pétrolier distillable entre 150 et 450˚C.

Enfin, l'invention concerne aussi un procédé pour prédire une propriété physique d'une coupe d'un échantillon pétrolier, dans lequel on simule l'effet d'une modification de l'intervalle de distillation de l'échantillon sur la propriété physique, en ne conservant que la partie du signal chromatographique correspondant à cet intervalle de distillation, et en calculant la propriété physique selon le procédé de l'invention.

D'autres caractéristiques et avantages du procédé selon l'invention, apparaîtront à la lecture de la description ci-après d'exemples non limitatifs de réalisations, en se référant aux figures annexées et décrites ci-après.

**Présentation succincte des figures**

- La figure 1 illustre le principe de reconstruction d'un chromatogramme en GC-2D.

- La figure 2 représente un chromatogramme 2D.

- La figure 3 est un exemple de prédiction de l'indice de cétane d'échantillons pétroliers.

- La figure 4 illustre un chromatogramme d'un gazole, effluent total d'une unité de raffinage. La figure 4A illustre le chromatogramme de l'échantillon total. Les traits en pointillé représentent la coupe pétrolière virtuelle 280-310°C. La figure 4B illustre le chromatogramme de la coupe réelle 280-310°C, obtenue par distillation physique de l'échantillon total.

**Description détaillée du procédé**

L'invention concerne un procédé pour déterminer les propriétés physiques d'un échantillon pétrolier. Ces propriétés physiques macroscopiques s'observent à l'échelle de l'échantillon. Il s'agit par exemple :

• des propriétés de combustion :

- indice d'octane (recherche ou moteur (RON ou MON), propriété mesurée sur les essences)

- indice de cétane (IC, propriété mesurée sur les gazoles)

- point de fumée (« Smoke Point », propriété mesurée sur les kérosènes)

- densité (à 15°C)

• des propriétés à froid :

- température limite de filtrabilité (TLF)

- point de trouble (« Cloud Point »)

- point d'écoulement (« Pour Point »)

- température de disparition des cristaux (« Freezing Point », propriété mesurée sur les kérosènes)

Selon le procédé, on détermine la valeur d'une propriété physicochimique donnée à partir de la composition moléculaire de l'échantillon, obtenue à partir d'une chromatographie en phase gazeuse bidimensionnelle (GC-2D). Le procédé comporte trois étapes majeures :

A- on réalise une chromatographie en phase gazeuse bidimensionnelle de l'échantillon pétrolier, et l'on construit un chromatogramme bidimensionnel ;

B- on détermine les concentrations des composés contenus dans l'échantillon, à partir d'une analyse du chromatogramme ;

C- on calcule les propriétés physicochimiques à partir de ces concentrations.

A- Chromatographie en phase gazeuse bidimensionnelle

La chromatographie en phase gazeuse bidimensionnelle (GC 2D) est une technique séparative particulièrement efficace pour effectuer des analyses moléculaires détaillées. Cette technique bien connue des spécialistes est décrite par exemple dans les brevets suivants : US 5,135,549 et US 5,196,039. Ces documents décrivent le principe du couplage en continu de deux colonnes de séparation différentes afin d'obtenir des chromatogrammes bidimensionnels. Une mise en oeuvre de chromatographie en phase gazeuse bidimensionnelle est décrite par exemple dans : L. Mondello, A. Lewis, K. Bartle, « Multidimensional gas chromatography », Ed. Wiley, 2002.

La chromatographie en phase gazeuse bidimensionnelle est une technique séparative dans laquelle tous les composés élués d'une première colonne sont successivement soumis à une séparation dans une deuxième colonne de sélectivité différente. Les deux colonnes sont connectées en série au moyen d'un modulateur qui est l'élément clé du dispositif. Cette interface échantillonne l'effluent de la première colonne sous forme d'impulsions chimiques et les transfère vers la deuxième colonne. La durée requise pour effectuer cette opération, appelée période de modulation, impose en général une deuxième séparation très rapide (quelques secondes) : les caractéristiques de la deuxième colonne sont choisies de telle sorte que chaque impulsion soit séparée durant la période de modulation.

Plus le composé a d'affinité avec la phase stationnaire, plus il mettra de temps à sortir de chacune des deux colonnes. A la sortie de la seconde colonne, les composés rencontrent un détecteur. Cet appareil mesure différentes propriétés physique du mélange gazeux sous forme d'une intensité en fonction du temps. Ce signal, nommé signal chromatogramme, ou 1 D brut, comporte un ensemble de pics, caractéristique de chaque constituant, dont la forme est fonction de l'intensité de la propriété mesurée. Chaque pic est nommé « pic d'élution » ou encore « pic chromatographique ». Le maximum de l'intensité correspondant à un pic est appelé temps de rétention. Le signal ainsi enregistré peut être de différente nature en fonction du détecteur utilisé. Les détecteurs (TCD, FID, SCD, NCD...) sont choisis en fonction du type d'application par l'homme de l'art. Certains détecteurs permettent de détecter quelques ppm (parties par million) d'un composant.

Le pic d'élution issu de la première colonne (1-A) est échantillonné périodiquement par le modulateur. Chaque fraction est focalisée puis injectée en continu dans la deuxième colonne. Le signal détecté, le signal 1D brut, correspond donc à une succession de séparations (matérialisées par des pics sur le signal) réalisées dans la deuxième dimension (1-B). En accolant ces chromatogrammes avec un offset, il est possible de reconstruire un signal dans les deux dimensions : le chromatogramme 2D.

L'image sur laquelle s'applique le procédé d'analyse est un chromatogramme en deux dimensions. Un tel chromatogramme est caractérisé par les éléments suivants :

- la période de modulation (*MC2*) : durée requise pour échantillonner l'effluent de la première colonne sous forme d'impulsions chimiques et les transférer vers la deuxième colonne.

- la coordonnée temporelle du premier point pris en compte (*TS* : *Time Start*). Ce point est choisit par l'utilisateur (les débuts de signaux sont parfois inexploitables).

- l'offset de deuxième colonne (*OC2*)

Ce chromatogramme est construit de la façon suivante :

- au cours de la chromatographie en phase gazeuse bidimensionnelle on enregistre un signal 1D brut, qui correspond au signal enregistré par le détecteur en sortie de la seconde colonne en fonction du temps (*t*). Un tel signal 1D brut (SB) est constitué d'un ensemble de points $P(t)$ ;

- les points de ce signal 1D brut (SB) ayant une coordonnée temporelle inférieure à *TS+OC2* sont retirés du signal ;

- le signal est découpé (*MOD*) en morceaux successifs de longueur *MC2*,

- ces morceaux sont concaténés (*CONC*) verticalement côte à côte pour former une image 2D, appelée chromatogramme 2D.

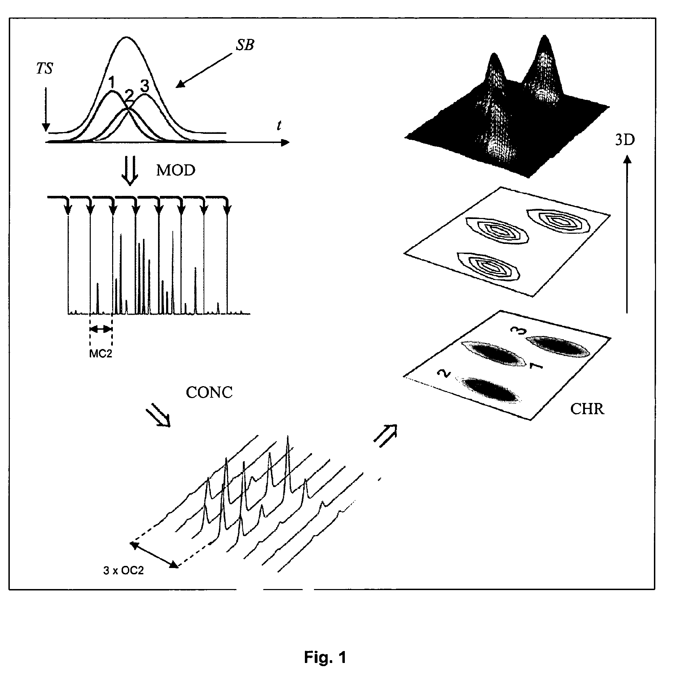

Ce chromatogramme 2D est la représentation la plus courante, il consiste donc en un ensemble de tranches (de largeur égale à la période de modulation *MC2*) de données brutes concaténées côte à côte. La figure 1 schématise le principe de reconstruction d'un chromatogramme en GC-2D, et un chromatogramme 2D (*CHR*) ainsi obtenu. Les deux axes du plan de séparation indiquant les coordonnées temporelles de séparation pour la première colonne en abscisse et pour la seconde colonne en ordonnée. Des pics chromatographiques forment alors des taches dont l'intensité est traduite par un dégradé de couleur. Cette représentation s'apparente à une image moléculaire de l'échantillon.

Effectuer ces opérations revient à appliquer les formules suivantes à chaque point $P(t)$ du signal 1D brut (SB) ayant une coordonnée temporelle supérieure à *TS+OC2* :

$$x = MC2 * floor\left(\left(t - TS - OC2\right)/MC2\right) + TS + OC2 \qquad (1)$$

$$y = \operatorname{mod}\left(\left(t - TS - OC2\right); MC2\right) \qquad (2)$$

Avec :

- $t$ : position temporelle du point $P(t)$ sur le signal 1D brut ;

- $x$, $y$ les coordonnées spatiales sur le chromatogramme 2D du point $P(t)$ ;

- *floor :* fonction qui retourne le plus grand entier qui n'est pas supérieur à l'argument ;

- mod : fonction qui renvoie le reste d'une division.

Notons que les relations exprimées ci-dessus ((1) et (2)), qui permettent de calculer les coordonnées spatiales ($x$, $y$) d'un point $P(t)$ à partir de ses coordonnées temporelles sont réversibles : $t = x + y$. Cette relation liant le temps absolu ($t$) et les coordonnées ($x$, $y$) d'un point sur l'image est respectée pour tout point apparaissant sur cette image (chromatogramme 2D), qu'il soit issu du signal 1D brut (SB) ou de la définition d'un polygone.

Cependant, les résultats issus d'une chromatographie en phase gazeuse bidimensionnelle (GC 2D) doivent être couplés à des méthodes d'analyses de données complexes.

B- Analyse du chromatogramme

Le préalable au calcul de propriétés macroscopiques, est d'obtenir la concentration des composés moléculaires séparés sur le chromatogramme bidimensionnel, de manière juste, répétable et non dépendante de l'opérateur.

Une technique particulièrement avantageuse pour interpréter une chromatographie en phase gazeuse bidimensionnelle et respectant ces conditions est décrite ci-après. Cette méthode comporte principalement trois étapes :

1- On délimite les taches du chromatogramme par des polygones, appelés blobs, qui correspondent à un ou plusieurs composants chimiques ;

2- On ajuste les polygones aux taches identifiées sur le chromatogramme ;

3- On détermine la composition moléculaire de l'échantillon en analysant les polygones.

*1- Délimitations des taches par des polygones sur ce chromatogramme*

Le chromatogramme 2D ainsi construit présente des taches dont l'intensité est traduite par un dégradé de couleur et qui représentent les pics chromatographiques. L'aire de ces taches est proportionnelle à la quantité d'un composé moléculaire spécifique. Une tache est une zone du chromatogramme 2D comportant au moins un pic d'élution. Ces taches sont appelées « blobs » par les spécialistes. Il convient donc, dans un premier temps, de délimiter ces zones. Cette délimitation forme un polygone.

Deux solutions peuvent être envisagées. Soit les polygones sont créés manuellement par une personne qui interprète l'image 2D, soit on applique un masque de polygones, c'est-à-dire un ensemble de polygones préalablement déterminés. Il peut s'agir d'un masque issu d'une analyse antérieur d'un soluté similaire par exemple.

Dans ce second cas, le masque doit être adapté à l'étude en cours. Le paragraphe précédent a montré comment l'image 2D (le chromatogramme 2D) était constituée de juxtapositions de segments du signal 1D brut (SB) tracés verticalement. La relation $t = x + y$ qui lie les points de l'image avec ceux du signal 1 D brut (SB) n'est valable que pour les points au centre des colonnes. Elle ne peut donc pas être appliquée directement. Un recalage horizontal doit être effectué. Il consiste à replacer chaque point saisi à la souris par l'utilisateur sur la colonne la plus proche :

$$x' = round\big((x - OC2 - TS)/MC2\big)* MC2 + OC2 + TS$$

avec *round* : fonction retournant l'entier le plus proche du nombre passé en argument.

A offset de deuxième colonne (*OC2*), coordonnée temporelle du premier point pris en compte (*TS*) et période de modulation (*MC2*) fixes, une bijection $t = x + y$ peut être définie. Elle associe à chaque instant un jeu de coordonnées dans l'image. Les polygones (blobs) sont donc stockés sous la forme suivante :

- {*tk*} : coordonnées temporelles sur le signal 1D brut (SB) des *k* sommets du polygone,

- *OC2* : offset de deuxième colonne à la création du polygone,

- *TS* : valeur de la coordonnée temporelle du premier point pris en compte à la création du polygone,

- *MC2* : période de modulation à la création du polygone.

Le stockage de la période de modulation permet de déterminer si un polygone est cohérent avec les paramètres d'acquisition d'une analyse (même période). Le stockage de l'offset de deuxième colonne et du *TS* permet de disposer d'au moins une configuration dans laquelle le polygone entier est visible sur l'image. Ce mode de stockage permet de réappliquer facilement un masque de polygone sur une nouvelle analyse même si l'offset utilisé est différent. Les fonctionnalités d'ajout, suppression, modification de sommets de polygone peuvent alors être facilement implémentées : les positions spatiales sont automatiquement converties en position temporelles. Cette structure de données permet alors :

- d'avoir une relation bijective entre l'image et le signal 1D brut (SB) ;

- d'être indépendant de l'offset choisi par l'utilisateur. Un masque de polygones défini avec un certain offset peut être appliqué sur une nouvelle analyse même si elle a un offset différent.

- de pouvoir utiliser les temps correspondants à l'intégration sur un signal 1D brut (SB) pour recaler les points sur l'image 2D.

*2- Ajustement des polygones à des taches identifiées sur le chromatogramme*

Grâce à la structure de données décrite précédemment, il est possible d'appliquer un masque de polygones sur une nouvelle analyse (ceci indépendamment de l'offset). L'objectif est ensuite de recaler les polygones sur une nouvelle analyse. C'est-à-dire de les caler sur des temps de début et de fin de pics d'élution sur les signaux 1D correspondant à l'intersection entre les colonnes de l'image et le polygone. L'ajustement des polygones est décomposé en trois étapes distinctes :

- détermination de signaux 1D (morceaux du signal 1D brut (SB)) correspondant à l'intersection entre un polygone et les colonnes de l'image ;

- détermination des pics d'élution sur ce signal 1D ;

- ajustement d'un polygone sur les temps de début et de fin des pics calculés précédemment.

*3- Détermination de la composition moléculaire de l'échantillon*

Les taches du chromatogramme 2D représentent un ensemble de pics chromatographiques. L'aire de ces taches est proportionnelle à la quantité d'un composé moléculaire spécifique.

Si toutes les coordonnées temporelles des intersections du polygone définissant le polygone avec les colonnes de l'image sont connues et ont été retraitées selon les principes énoncés au paragraphe précédent, alors les aires peuvent être calculées simplement.

Les traitements ne sont effectués pour chaque polygone que si la période de modulation courante est identique à celle mémorisée dans le polygone. Les calculs d'aires sont tous effectués avec un offset calculé de sorte que le polygone soit au centre de l'image. Cela permet d'obtenir pour chaque analyse des fichiers de résultat indépendants de l'offset de deuxième colonne.

C- Calcul des propriétés physicochimiques

Pour décrire le procédé de détermination des propriétés physicochimiques macroscopiques d'un échantillon pétrolier, à partir de l'analyse quantitative de cet échantillon par GC-2D, on considère l'indice de cétane (IC) et des échantillons de gazoles.
On réalise donc une analyse quantitative de vingt échantillons de gazole par GC 2D. Ces analyses fournissent, pour chaque échantillon de gazole, la concentration des composés moléculaires constituants ces échantillons.
Puis on définit une relation *f*, permettant de déterminer l'indice de cétane (*IC*) d'un échantillon, en fonction de la concentration (*C_i*) de ces différents composés moléculaires i:

$$IC = f(C_i) = \sum_{i=1}^{n} IC_i^{ajust} . C_i$$

avec :

- *n* le nombre de composés moléculaires séparés par chromatographie

- $IC_i^{ajust}$ est une valeur ajustée de l'indice de cétane pour un composé *i*.

Pour déterminée les valeurs de $IC_i^{ajust}$, on procède de la façon suivante :

- on initiale ces valeurs à partir de $IC_i^{ini}$, la valeur connue de l'indice de cétane pour un composé *i*, indépendamment de sa concentration. Par exemple, l'indice de cétane du N-C16 est de 100. Ces valeurs initiales sont données par la littérature, ou des contraintes chimiques (par exemple l'indice de cétane des normales paraffines est supérieur à l'indice de cétane des iso-paraffines). Le tableau suivant présente quelques valeurs initiales :

| Noms des composants | $IC_i^{ini}$ |
|---|---|
| C13 ISO PARAFFINES | 0,1558 |
| C14 ISO PARAFFINES | 0,6611 |
| C15 ISO PARAFFINES | 0,6992 |
| C16 ISO PARAFFINES | 0,4021 |
| C17 ISO PARAFFINES | 0,3354 |
| C18 ISO PARAFFINES | 0,8134 |
| C19 ISO PARAFFINES | 0,8515 |
| C20 ISO PARAFFINES | 0,8896 |

- on analyse des échantillons de gazole par GC 2D, dont on mesure l'indice de cétane.
- on calcule l'indice de cétane à l'aide de la relation *f*.

- on ajuste les valeurs de $IC_i^{ini}$, par une méthode de type moindres carrées en intégrant des contraintes chimiques.

Le tableau suivant présente les valeurs initiales ajustées :

| Noms des composants | $IC_i^{ajust}$ |
|---|---|
| C13 ISO PARAFFINES | 0.3894 |
| C14 ISO PARAFFINES | 0.4132 |
| C15 ISO PARAFFINES | 0.437 |
| C16 ISO PARAFFINES | 0.4608 |
| C17 ISO PARAFFINES | 0.4846 |

(suite)

| Noms des composants | $IC_i^{ajust}$ |
|---|---|
| C18 ISO PARAFFINES | 0.5084 |
| C19 ISO PARAFFINES | 0.5322 |
| C20 ISO PARAFFINES | 0.556 |

On réalise ce type d'ajustement sur un grand nombre d'échantillons de gazole de façon à déterminer les valeurs ajustées $IC_i^{ajust}$ des composés hydrocarbonés susceptibles d'être présent dans un échantillon pétrolier.

De cette façon, on obtient un modèle explicite, composé de la fonction *f* et des valeurs ajustées $IC_i^{ajust}$.

Bien entendu, on peut choisir une autre fonction *f*, et réaliser un nouvel ajustement de façon à obtenir un nouveau modèle.

Il est important de noter que ce modèle est valable pour tout type de Gazole, et qu'il peut prendre en compte un post-traitement sur le chromatogramme (par exemple une coupe pétrolière).

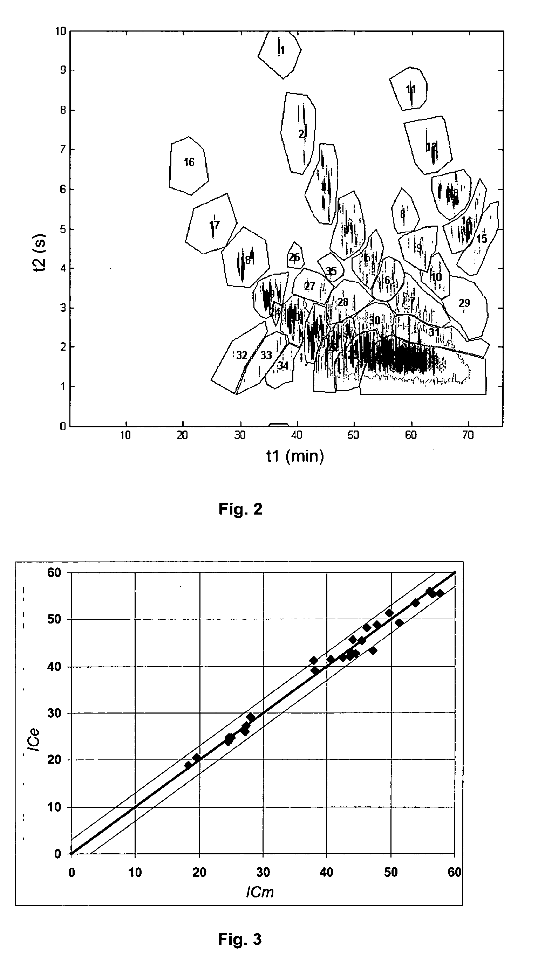

Ainsi, après avoir réalisé une analyse GC 2D sur un échantillon pétrolier, et construit un chromatogramme tel que celui de la figure 2, on définit des blobs (figure 2) dont on déduit les concentrations de chaque composé moléculaire. La figure 2 représente un chromatogramme 2D obtenu pour la séparation de composés azotés contenus dans un échantillon de distillat moyen. Les deux axes du plan de séparation indiquant les coordonnées temporelles de séparation pour la première colonne en abscisse et pour la seconde colonne en ordonnée. Des pics chromatographiques forment alors des taches dont l'intensité est traduite par un dégradé de couleur. Cette représentation s'apparente à une image moléculaire de l'échantillon.

L'utilisateur lance ensuite les calculs et visualise :
- les propriétés finales de chaque composant après coupe :

| Noms des composants | Nom de la famille | Temps de rétention (s) | Température d'ébullition (˚C) | Indice de Cétane |
|---|---|---|---|---|
| C13 ISO PARAFFINES | ISO PARAFFINES | 1923,36 | 217,413005 | 0 |
| C14 ISO PARAFFINES | ISO PARAFFINES | 2663,772 | 250,2371826 | 2,81323018 |
| C15 ISO PARAFFINES | ISO PARAFFINES | 3003,948 | 264,7309332 | 5,769673326 |
| C16 ISO PARAFFINES | ISO PARAFFINES | 3324,09 | 278,7801639 | 4,72741323 |
| C17 ISO PARAFFINES | ISO PARAFFINES | 3684,18 | 294,4823919 | 3,671947351 |
| C18 ISO PARAFFINES | ISO PARAFFINES | 3884,148 | 302,9741152 | 4,285301098 |
| C19 ISO PARAFFINES | ISO PARAFFINES | 4224,282 | 318,0831418 | 4,763893579 |
| C20 ISO PARAFFINES | ISO PARAFFINES | 4644,42 | 336,4711929 | 2,965664768 |

- les propriétés physiques pour chaque famille après coupe :

| Nome de Famille | Indice de Cétane |
|---|---|
| DI-NAPHTHÈNES | 3,159854543 |
| MONO-NAPHTHÈNES | 19,45162568 |
| MONO-AROMATIQUES | 11,4218426 |
| DI-AROMATIQUES | 8,952249288 |
| TRI-AROMATIQUES | 1,43685633 |
| TETRA-AROMATIQUES | 0 |

(suite)

| Nome de Famille | Indice de Cétane |
|---|---|
| NAPHTO-MONO-AROMATIQUES | 0 |
| NAPHTO-DI-AROMATIQUES | 0 |
| NAPHTO-TRI-AROMATIQUES | 0 |
| N-PARAFFINES | 19,15360108 |
| ISO-PARAFFINES | 29,43300901 |

- les propriétés physiques de l'échantillon après coupe

| | |
|---|---|
| somme des surfaces de blobs | 100 |
| fraction carbone | 1526,958407 |
| poids moléculaire | 21444,61929 |
| densité (15˚C) | 93,00903854 |
| pression de vapeur (hPa) | 93,00903854 |
| **indice de cétane** | 93,00903854 |
| RON | **93,00903854** |
| MON | 93,00903854 |
| carbone oléfinique | 0 |
| carbone aromatique | 1196,754823 |
| carbone paraffine | 7315,742657 |
| carbone naphténique | 788,4063742 |
| carbone | 7949,283561 |
| hydrogène | 1351,620292 |
| Sulfure | 0 |
| oxygène | 0 |
| nitrogène | 0 |

La figure 3 illustre un exemple de prédiction de l'indice de cétane d'échantillons pétroliers. L'axe horizontal indique l'indice de cétane mesuré (*ICm*) sur moteur CFR, et l'axe vertical indique l'indice de cétane estimé (*ICe*) à partir des données issues de la GC 2D.

De façon générale, selon l'invention, on calcule les propriétés physiques macroscopiques à partir de l'aire des différents blobs d'un chromatogramme 2D, ces aires étant représentatives de la quantité d'un composé, et peuvent être directement reliées aux concentrations des composés composant l'échantillon pétrolier.

On calcule une propriété macroscopique $P_m$ à partir des quantités, au moyen d'une relation *f* définie par :

$$P_m = f(\alpha_i, Q_i, n)$$

avec :

$P_m$ : la propriété macroscopique

$n$ : le nombre de composés moléculaires séparés par chromatographie

$\alpha_i$ : un coefficient préalablement calibré et associé au composé *i*

$Q_i$ : la quantité du composé *i* déterminée par chromatographie (la concentration par exemple, ou l'aire du blobs)

*Définition de la relation f*

La relation *f* peut être définie par exemple par la relation suivante :

$$f\left(\alpha_i, Q_i, n\right) = \sum_{i=1}^{i=n} \alpha_i . Q_i$$

On exprime alors le fait que la propriété macroscopique d'un échantillon pétrolier est une combinaison linéaire des concentrations de chacun des composés moléculaires de cet échantillon.

*Calibration de la relation f*

Cette relation est ensuite calibrée. Pour ce faire on réalise les étapes suivantes :

- on détermine des quantités de composés présents dans un second mélange (en général, un ensemble d'échantillons pétrolier est collecté) contenant au moins les n composés moléculaires, au moyen d'une chromatographie en phase gazeuse bidimensionnelle ;

- on mesure la propriété macroscopique $P_m$ de ce second mélange ;

- on initialise les coefficients $\alpha_i$ en leur affectant une valeur connue de propriété physiques macroscopique pour un échantillon ne contenant que le composé *i*;

- on calcule la propriété physique macroscopique au moyen de la fonction *f* ;

- on modifie les coefficients $\alpha_i$ de façon à minimiser la différence entre la valeur de la propriété physique macroscopique calculée, et la valeur de la propriété physique macroscopique mesurée et en intégrant des contraintes chimiques.

Les coefficients $\alpha_i$ du modèle peuvent être déterminés par une méthode classique type moindres carrés avec contraintes, de façon à minimiser la différence entre la valeur de la propriété physique calculée, et la valeur de la propriété macroscopique mesurée directement sur l'échantillon.

Selon l'exemple de réalisation décrit précédemment, on peut écrire :

$$IC_i^{ajust} = \alpha_i \text{ et } C_i = Q_i$$

*Utilisation de la relation f*

Une fois le modèle défini et calibré, c'est-à-dire la relation *f* définie et les paramètres calibrés, l'utilisateur dispose d'un modèle explicite, défini pour des composants chimiques. Il n'y a donc pas de problèmes de sur-apprentissage ou de sur-paramétrage.

On peut donc utiliser ce modèle pour déterminer les propriétés macroscopiques d'un échantillon pétrolier, après en avoir analysé la composition moléculaire, en appliquant la formule.

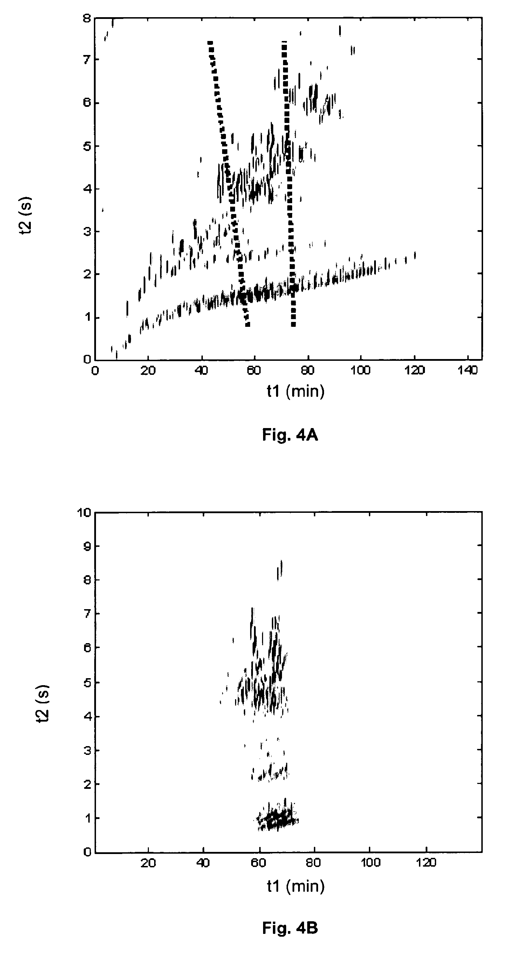

On peut également estimer les propriétés macroscopiques pour une coupe pétrolière quelconque, en supprimant certaines parties du chromatogramme (coupe). On convertit le temps de la première dimension du chromatogramme en température via la connaissance de températures d'ébullition de composés de référence (en général n-paraffines), puis on ne conserve que la partie du signal chromatographique correspondant à l'intervalle de coupe. On calcule alors la propriété macroscopique au moyen de la relation *f*. La figure 4 illustre un chromatogramme d'un gazole, effluent total d'une unité de raffinage. La figure 4A illustre le chromatogramme de l'échantillon total. Les traits en pointillé représentent la coupe pétrolière virtuelle 280-310˚C. La figure 4B illustre le chromatogramme de la coupe réelle 280-310˚C, obtenue par distillation physique de l'échantillon total. On simule ainsi l'effet d'une modification de l'intervalle de distillation d'un échantillon sur la propriété macroscopique.

On peut également estimer l'évolution de ces propriétés suite à des transformations chimiques de l'échantillon pétrolier, en simulant l'effet d'une transformation chimique d'une famille de composés sur les propriétés macroscopiques.

**Avantages**

L'objet de la présente invention permet de réaliser directement les calculs de propriétés à partir de la composition chimiques d'un échantillon analysé par GC*GC. La chromatographie bidimensionnelle permettant de relier par une relation bi-univoque la propriété d'un blob à sa concentration, les propriétés physiques macroscopiques prédites sont donc indépendantes de toute base de calibration. Elles sont donc extrapolables, et permettent donc, non seulement de prédire les propriétés macroscopiques d'un échantillon, mais également d'estimer ces propriétés pour une coupe pétrolière quelconque (distillation), et d'estimer l'évolution de ces propriétés suite à des transformations chimiques de l'échantillon pétrolier (hydrogénation). La méthode est particulièrement adaptée aux échantillons pétroliers distillables entre 150˚C et 450˚C.

La méthode est suffisamment générique : tout type de propriété peut être calculé. La formule de calcul peut être linéaire ou non.

**Revendications**

1. Procédé de détermination d'au moins une propriété physique d'un mélange de composés moléculaires tel qu'un échantillon pétrolier, **caractérisé en ce que** l'on réalise les étapes suivantes :

   - on détermine les quantités de *n* composés présents dans ledit mélange, au moyen d'une chromatographie en phase gazeuse bidimensionnelle ;
   - on détermine ladite propriété physique à partir desdites quantités, au moyen d'une relation *f* préalablement calibrée reliant ladite propriété physique auxdites quantités.

2. Procédé selon la revendication 1, dans lequel ladite relation *f* est définie par :

$$P_m = f(\alpha_i, Q_i, n)$$

   où $P_m$ est la propriété physique, *n* est le nombre de composés moléculaires séparés par ladite chromatographie, $\alpha_i$ est un coefficient à calibrer et associé à un composé *i*, et $Q_i$ est la quantité du composé *i* déterminée par ladite chromatographie, et dans lequel on calibre ladite relation *f* en réalisant les étapes suivantes :

   - on détermine des quantités de composés présents dans au moins un second mélange contenant au moins les *n* composés moléculaires, au moyen d'une chromatographie en phase gazeuse bidimensionnelle ;
   - on mesure ladite propriété physique $P_m$ dudit second mélange ;
   - on initialise les coefficients $\alpha_i$ en leur affectant une valeur connue de propriété physique pour un échantillon ne contenant que le composé *i*;
   - on calcule ladite propriété physique au moyen de ladite fonction *f* ;
   - on modifie les coefficients $\alpha_i$ de façon à minimiser une différence entre la valeur de la propriété physique calculée, et la valeur de la propriété physique mesurée.

3. Procédé selon l'une des revendications précédentes, dans lequel la relation *f est* définie par :

$$f(\alpha_i, Q_i, n) = \sum_{i=1}^{i=n} \alpha_i . Q_i$$

4. Procédé selon l'une des revendications précédentes, dans lequel on réalise la chromatographie en phase gazeuse bidimensionnelle, au moyen des étapes suivantes :

   - on enregistre un signal chromatographique comportant des pics chromatographiques ;
   - on génère un chromatogramme en deux dimensions dont chaque colonne correspond à une portion dudit signal chromatographique, lesdits pics chromatographiques formant des taches sur ledit chromatogramme ;

- on délimite lesdites taches à l'aide de polygones ;
- on ajuste lesdits polygones :
- en identifiant des portions dudit signal chromatographique comprises entre deux intersections dudit polygone avec des colonnes dudit chromatogramme ;
- en déterminant des temps de début, des temps de fin et des maximums de pics chromatographiques présents sur lesdites portions ;
- en déplaçant lesdits points d'intersection en fonction desdits temps de début, desdits temps de fin et desdits maximums de pics chromatographiques ;
- on détermine une quantité d'au moins un composé moléculaire en calculant l'aire dudit polygone ainsi ajusté.

5. Procédé selon la revendication 4, dans lequel on détermine la quantité d'un composé en estimant sa concentration à partir de l'aire dudit polygone.

6. Procédé selon l'une des revendications précédentes, dans lequel la propriété physique est choisie parmi les propriétés suivantes :

- propriétés de combustion : indice d'octane, indice de cétane, point de fumée et densité ;
- propriétés à froid : température limite de filtrabilité, point de trouble, point d'écoulement, température de disparition des cristaux.

7. Procédé selon l'une des revendications précédentes, dans lequel ledit mélange est un échantillon pétrolier distillable entre 150 et 450˚C.

8. Procédé selon l'une des revendications 1 à 7, pour prédire une propriété physique d'une coupe d'un échantillon pétrolier, dans lequel on simule l'effet d'une modification de l'intervalle de distillation de l'échantillon sur la propriété physique, en ne conservant que la partie du signal chromatographique correspondant audit intervalle de distillation, et en calculant ladite propriété physique au moyen du procédé selon l'une des revendications 1 à 7.

**Fig. 1**

**Fig. 2**

**Fig. 3**

Fig. 4A

Fig. 4B

| | Europäisches Patentamt<br>European Patent Office<br>Office européen des brevets | **RAPPORT DE RECHERCHE EUROPEENNE** | Numéro de la demande<br>EP 09 29 0305 |
|---|---|---|---|

## DOCUMENTS CONSIDERES COMME PERTINENTS

| Catégorie | Citation du document avec indication, en cas de besoin,<br>des parties pertinentes | Revendication<br>concernée | CLASSEMENT DE LA<br>DEMANDE (IPC) |
|---|---|---|---|
| X | WO 2006/055499 A (EXXONMOBIL RES & ENG CO [US]; WANG FRANK CHENG-YU [US]; ZHANG LEI [US]) 26 mai 2006 (2006-05-26)<br>* abrégé *<br>* alinéa [0056] - alinéa [0067] *<br>* tableaux 4-6 *<br>* figure 3 *<br>----- | 1 | INV.<br>G01N30/46<br>G01N30/86<br>G01N33/28<br><br>ADD.<br>G01N30/02 |
| X | VENDEUVRE C ET AL: "Characterisation of middle-distillates by comprehensive two-dimensional gas chromatography (GCxGC): A powerful alternative for performing various standard analysis of middle-distillates"<br>JOURNAL OF CHROMATOGRAPHY, ELSEVIER SCIENCE PUBLISHERS B.V. AMSTERDAM, NL,<br>vol. 1086, no. 1-2,<br>9 septembre 2005 (2005-09-09), pages 21-28, XP004995136<br>ISSN: 0021-9673 | 1,6,7 | |
| Y | * abrégé *<br>* page 21, colonne 1, ligne 1 - ligne 5 *<br>* page 23, colonne 2, ligne 5 - ligne 11 *<br>* page 25, colonne 2, dernier alinéa - page 27, colonne 2, dernier alinéa *<br>* figures 4-7 *<br>----- | 2-5,8 | DOMAINES TECHNIQUES<br>RECHERCHES (IPC)<br><br>G01N |
| Y | FR 2 774 768 A (INST FRANCAIS DU PETROLE [FR]) 13 août 1999 (1999-08-13)<br>* abrégé *<br>* page 15, ligne 1 - ligne 6 *<br>* page 23, ligne 10 - page 4, ligne 19 *<br>-----<br>-/-- | 2,3,8 | |

Le présent rapport a été établi pour toutes les revendications

| Lieu de la recherche | Date d'achèvement de la recherche | Examinateur |
|---|---|---|
| Munich | 28 mai 2009 | Bravin, Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................................
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 09 29 0305

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | BEENS JAN ET AL: "Quantitative Aspects of Comprehensive Two-Dimensional Gas Chromatography (GCxGC)" JOURNAL OF SEPARATION SCIENCE, WILEY, DE, vol. 21, 1 janvier 1998 (1998-01-01), pages 47-54, XP002440106 ISSN: 1615-9306 * page 48, colonne 1, dernier alinéa - page 51, colonne 1, dernier alinéa * * figure 5 * ----- | 4,5 | |
| A | BEENS JAN, BRINKMANN UDO A TH: "The role of gas chromatography in compositional analyses in the petroleum industry" TRENDS IN ANALYTICAL CHEMISTRY, vol. 19, no. 4, avril 2000 (2000-04), pages 260-275, XP002517805 ELSEVIER * le document en entier * ----- | 1-8 | |
| P,Y | EP 1 953 545 A (IFP [FR]) 6 août 2008 (2008-08-06) * alinéas [0012], [0013] * * revendication 1 * ----- | 4,5 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 28 mai 2009 | Bravin, Michel |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 29 0305

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-05-2009

| Document brevet cité au rapport de recherche | | Date de publication | Membre(s) de la famille de brevet(s) | | Date de publication |
|---|---|---|---|---|---|
| WO 2006055499 | A | 26-05-2006 | AU | 2005306342 A1 | 26-05-2006 |
| | | | AU | 2005306674 A1 | 26-05-2006 |
| | | | AU | 2005306675 A1 | 26-05-2006 |
| | | | AU | 2005306677 A1 | 26-05-2006 |
| | | | CA | 2585194 A1 | 26-05-2006 |
| | | | CA | 2585198 A1 | 26-05-2006 |
| | | | CA | 2586483 A1 | 26-05-2006 |
| | | | EP | 1812791 A1 | 01-08-2007 |
| | | | EP | 1824948 A1 | 29-08-2007 |
| | | | EP | 1817578 A1 | 15-08-2007 |
| | | | EP | 1841844 A2 | 10-10-2007 |
| | | | JP | 2008520973 T | 19-06-2008 |
| | | | JP | 2008520763 T | 19-06-2008 |
| | | | JP | 2008520974 T | 19-06-2008 |
| | | | JP | 2008520770 T | 19-06-2008 |
| | | | US | 2008206878 A1 | 28-08-2008 |
| | | | US | 2008163672 A1 | 10-07-2008 |
| | | | US | 2008116108 A1 | 22-05-2008 |
| | | | WO | 2006055306 A1 | 26-05-2006 |
| | | | WO | 2006055500 A1 | 26-05-2006 |
| | | | WO | 2006055502 A1 | 26-05-2006 |
| | | | WO | 2006055901 A2 | 26-05-2006 |
| FR 2774768 | A | 13-08-1999 | US | 6275775 B1 | 14-08-2001 |
| EP 1953545 | A | 06-08-2008 | CN | 101236184 A | 06-08-2008 |
| | | | FR | 2911962 A1 | 01-08-2008 |
| | | | JP | 2008185586 A | 14-08-2008 |
| | | | US | 2008180447 A1 | 31-07-2008 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5699269 A
- US 6275775 B
- US 5135549 A
- US 5196039 A

**Littérature non-brevet citée dans la description**

- **Jenkins G.J. ; Mc Taggart N.G. ; Watkin B.L.H.** GLC for on-stream Octane number Rating of stabilized catalitic reformates. *Gas Chromatography, Ed. Inst. Petr.,* 1968, 185-198
- **Kelly J.J. ; Barlow C.H. ; Jinguji T.M. ; Callis J.B.** Prediction of octane numbers from near-infrared spectral features in the range 660-1212 nm. *Analytical Chemistry,* 1989, vol. 61 (4), 313
- **Espinosa A. ; Lambert D. ; Valleur M.** Use NIR technology to optimize plant operation. *Hydrocabon Processing,* Février 1986
- **J.P.Durand ; Y. Boscher ; N. Petroff ; M. Berthelin.** *J. Chromatogr.,* 1987, vol. 395, 229
- **L. Mondello ; A. Lewis ; K. Bartle.** Multidimensional gas chromatography. 2002